Europäisches Patentamt

European Patent Office

Office européen des brevets

(19)

(11) Publication number: **0 215 351 B1**

# EUROPEAN PATENT SPECIFICATION

(45) Date of publication of patent specification: **10.04.91**

(21) Application number: 86111905.5

(22) Date of filing: 28.08.86

(51) Int. Cl.⁵: **C07C 45/46**, C07C 49/76, C07C 49/788, C07C 49/825, C07C 49/83, C07C 49/84

(54) **Process for producing acetyl-substituted aromatic compound.**

(30) Priority: 31.08.85 JP 191521/85

(43) Date of publication of application:
25.03.87 Bulletin 87/13

(45) Publication of the grant of the patent:
10.04.91 Bulletin 91/15

(84) Designated Contracting States:
DE FR GB IT NL

(56) References cited:
EP-A- 0 069 597          EP-A- 0 170 483
EP-A- 0 196 805          EP-A- 0 203 276
FR-A- 2 348 181          US-A- 3 234 286

CHEMICAL ABSTRACTS, vol. 104, no. 13,
March 1986, Columbus, OH (US); p. 685, no.
109223s & JP-A-60 188343

J.A.C.S., vol. 61, July 1939, pages 1795-1796;
J.H. SIMONS et al.: "Hydrogen fluoride as a
condensing agent. VII. The acylation of ar-
omatic compounds"

(73) Proprietor: MITSUBISHI GAS CHEMICAL COM-
PANY, INC.
5-2, Marunouchi 2-chome Chiyoda-Ku
Tokyo, 100(JP)

(72) Inventor: Fujiyama, Susumu
522-65, Kamitomii
Kurashiki-shi(JP)
Inventor: Matsumoto, Shunichi
1168-3, Tanoue
Kurashiki-shi(JP)
Inventor: Yanagawa, Tatsuhiko
1987, Nakashima
Kurashiki-shi(JP)

(74) Representative: Patentanwälte Grünecker,
Kinkeldey, Stockmair & Partner
Maximilianstrasse 58
W-8000 München 22(DE)

## Description

This invention relates to a continuous process for producing an acetyl-substitued aromatic compound which comprises:

obtaining an acetyl fluoride by the reaction of acetic anhydride with a substantially anhydrous hydrogen fluoride and separating formed acetyl fluoride by distillation,

making an aromatic compound selected from alkylbenzenes, alkyl-naphtalenes, phenols, naphtols or aromatic ethers react with the separated acetyl fluoride in the presence of substantially anhydrous hydrogen fluoride as a catalyst at a temperature of from $0^\circ$ - $70^\circ$ C,

thermally decomposing the resulting complex compound between the acetyl-substituted aromatic compound and hydrogen fluoride at a temperature of $40^\circ$C or higher.

It is disclosed in Japanese Patent Application Kokai (Laid-open) No. 135756/79 to produce a 2-alkyl-6-acylnaphthalene by acylating a 2-alkynaphthalene with an acid fluoride in the presence of boron fluoride as a catalyst.

It is described in the above-mentioned invention that the presence of boron fluoride as a catalyst component is essential and the absence of boron fluoride results in low yield of the acylation product. However, according to the experiment of the present inventors, it has been found that when boron fluoride is used as a catalyst component in the reaction of aromatic compound with acetyl fluoride, it is difficult to decompose the resulting complex compound on accound of the instability of aromatic keton of the reaction product.

EP-A-196 805 discloses acylation of a naphthalene compound in a batch or semi-continuous manner by using hydrogen fluoride and an acylating agent.

EP-A-203 276 discloses a process for selectively acylating of aromatic compounds wherein an alkyl or aryl substituted aromatic compound is dissolved in hydrogen fluoride and reacted with an acylating agent.

EP-A-69 597 discloses the preparation of p-phenoxybenzoyle ketone in a batch manner by reacting diphenyl ether and an acylating agent and converting the resulting p-phenoxybenzoyl ketone into p-phenoxybenzoic acid.

EP-A-170 483 discloses to produce 4-hydroxy-acetophenone in a batch wise manner by Friedel-Crafts acetylation of phenol, using hydrogen fluoride as catalyst and acetic acid as acylating agent.

The object of the present invention is to conduct a process for producing an acetyl substituted aromatic compound continuously.

Accordingly, the present inventors have made extensive studies to attain a process for producing an acetyl-substituted aromatic compound with higher efficiency including the steps of the decomposition of the complex compound and the recovery of the catalyst component.

The object of the present invention has been achieved in obtaining acetyl fluoride by making the molar ratio of acetyle fluoride to starting aromatic compound 1 or below, and making an excess of acetic anhydride of 5 mol % or below react with hydrogen fluoride.

It has been found that, in the reaction of an aromatic compound with acetyl fluoride, even when hydrogen fluoride is used as a catalyst they intended acetyl-substituted aromatic compound can be obtained in excellent yield and further the complex compound form can be readily decomposed and the hydrogen fluoride catalyst can be easily recovered.

This invention has been accomplished on the basis of the above findings.

Thus, this invention provides a continuous process for producing an acetyl-substituted aromatic compound which comprises making an aromatic compound including an 2-alkylnaphthalene react with acetyl fluoride in the presence of substantially anhydrous hydrogen fluoride as a catalyst.

The aromatic compounds used as the starting material in this invention include alkylbenzenes such as toluene, xylene, trimethylbenzene, ethylbenzene, cumene, and butylbenzene; naphthalene and alkylanphthalenes such as methylnaphthalene; phenols and naphthols; and further aromatic ethers such as anisole and phenyl ether. Particularly preferred are compounds in which the para position to the substituent in the aromatic ring is vacant and naphthalenes having a substituent in the 2-position.

Acetyl fluoride as the other starting material, can be obtained by mixing acetic anhydride with hydrogen fluoride to produce acetyl fluoride according to the following equation (1) and separating the free acid simultaneously formed.

$$(CH_3CO)_2O + HF \rightarrow CH_3COF + CH_3COOH \ ........ (1)$$

It is essential here that the reaction (1) mentioned above should be carried out at a slight excess of acetic anhydride. Thus, when hydrogen fluoride is in excess of the equivalent, the maximum azeotropic mixture combined hydrogen fluoride with the acid, which leads to the loss of hydrogen fluoride, is formed to become inseparable and further the acid formed is contaminated with fluorine, so that special treatments are required to remove it. when acetic anhydride is in excess, such difficulties do not occur and hydrogen fluoride can be quantitatively recovered as acetyl fluoride. However, there is no need of large excess and the ratio of excess acetic anhydride to hydrogen fluoride may be 5 mol% or below.

The apparatus used for generating acetyl fluoride may be a conventional distillation column having a number of plates necessary for fractionating acetyl fluoride and the free acid. Acetic anhydride and hydrogen fluoride are fed to the appropriate plate of the distillation column as a mixture or separately, the column bottom is heated up to the boiling point of the acid and the column top is given an appropriate reflux. Such a conventional distillation operation makes it possible to recover pure acetyl fluoride from the column top and an acid containing no fluorine from the column bottom.

Since the reaction proceeds at a high rate, virtually no residence time is necessary. The reaction can be conducted under an ordinary or an applied pressure, for example 1 kg/cm$^2$G, and either flow operation or batchwise operation may be used. The difference of boiling point between acetyl fluoride and the acid formed is so large that the two can be easily separated.

The acetyl fluoride thus formed is used for acetylation as the acetylating agent. The molar ratio of acetyl fluoride to the starting aromatic compound is 1 or below, 0.9 to 0.5 being particularly preferable. The presence of excess acetyl fluoride decreases the overall reaction rate (i.e. the space time yield of the acetylation product).

As the catalyst, a substantially anhydrous hydrogen fluoride is used. Its water content is preferably 5% or below because the presence of water in hydrogen fluoride causes the rapid decrease of catalytic activity. In order to obtain a sufficient reaction rate, the molar ratio of hydrogen fluoride to be used relative to the acetylating agent is 5 or above, preferably in the range of 10 to 20. Hydrogen fluoride used in a molar ratio of 20 or above gives little additional effect and hence is not advantageous from the economical viewpoint of the process.

The reaction temperature of acetylation is 0 to 70°C, preferably 10 to 50°C. Since the reaction rate increases as the temperature is increased but also the side reaction increases, an optimum temperature is selected from the above-mentioned range depending upon the starting material used. When the starting compound has a high melting point and further is insoluble in hydrogen fluoride as in the case of aromatic hydrocarbons, it is effective to use a suitable solvent in order to make the reaction proceed smoothly. Preferable solvents are those which can dissolve the starting compound well, are chemically inert under reaction conditions, and have a good compatibility also with the reaction liquid formed. They include, for example, benzene or halogenated hydrocarbons such as chlorobenzene, dichloromethane, dichloroethane and freon. Particularly, benzene is the most suitable solvent in the present process because it is not only substantially inert under the present reaction conditions but also favorable as the solvent in the step of recovering the catalyst from the reaction mixture.

The amount of the solvent to be used in the reaction is not specifically limited. The amount of 0.5 mole or below per mole of the starting compound is usually sufficient.

Although the reaction pressure is varied depending on the reaction temperature, usually it ranges from an ordinary pressure to a slightly elevated pressure of up to 2 kg/cm$^2$G.

The reaction proceeds in a homogeneous liquid phase or, according to circumstances, in two-liquid phases consisting of a starting aromatic compound phase and a catalyst phase, so that there is no need of vigorous stirring. Since the reaction is slightly exothermic, a reactor provided with heat removal equipment is used as required.

The acetylation reaction liquid thus obtained is a solution of an aromatic ketone, the acetylation product, in hydrogen fluoride. On heating the reaction liquid, the affinity between the reaction product and hydrogen fluoride is broken and hydrogen fluoride can be easily vaporized and separated.

It is necessary to conduct the above-mentioned catalyst recovery operation as rapidly as possible in order to avoid the thermal degradation of the reaction product. For this purpose, the catalyst recovery operation is preferably conducted in a flow operation using a multistage gas-liquid contact apparatus (i.e. a distillation column). For catalyst recovery, heating at a temperature of 40°C or higher, particularly 40 to 100°C, is necessary. The decomposition column is preferably fed with an amount of heat which is in excess of that necessary for vaporizing hydrogen fluoride fed to the column. It is advantageous in the process to conduct the catalyst recovery operation under atmospheric pressure or a slightly increased pressure of 2 kg/cm$^2$G or below. In order to make the thermal decomposition of the complex compound between the acetyl-substituted aromatic compound and hydrogen fluoride proceed smoothly, the decomposition is preferably conducted by heating the complex compound under reflux using as a diluent a substance which has a boiling point such that it is easily separable from hydrogen fluoride, has a good compatibility with the reaction product, namely the acetyl-substituted aromatic compound, and with hydrogen fluoride, and is inert to hydrogen fluoride. Examples of such diluent used include aromatic

3

compounds such as benzene and chlorobenzene. Particularly, benzene is the most preferable.

According to this invention, aromatic compounds can be acetylated in a simple operation under low reaction pressure, and hydrogen fluoride used as the catalyst can be completely recovered and recycled. So this invention is of great industrial advantage.

The accompanying drawing is a flow diagram showing the acetylation process of this invention.

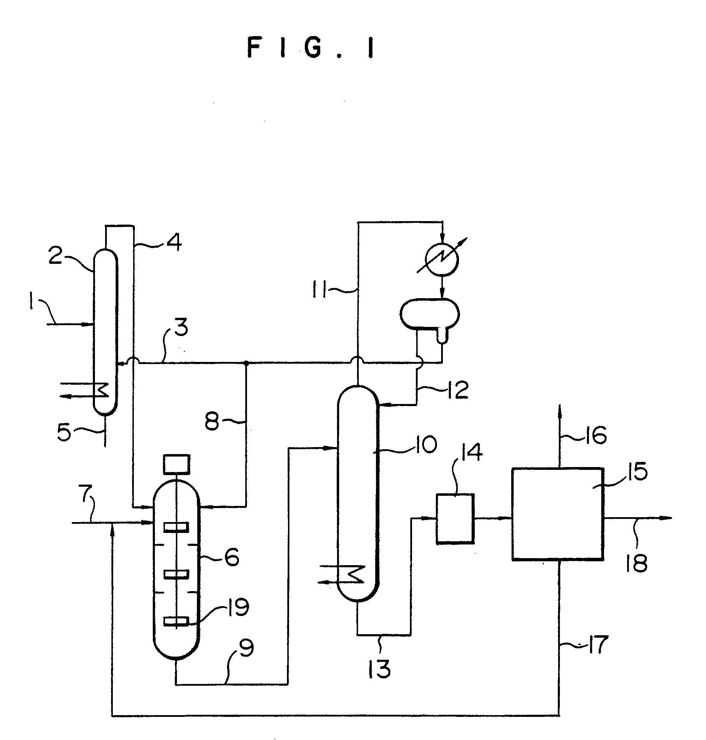

The process for acetylating an aromatic compound according to this invention is illustrated below with reference to Fig. 1.

In Fig. 1, acetic anhydride is fed to the middle plate of an acetyl fluoride generating apparatus 2 through pipe 1 and contacted there with heating with hydrogen fluoride introduced through a pipe 3. The acetyl fluoride formed is distilled out through an outlet pipe 4 and acetic acid is withdrawn through a pipe 5. The acetyl fluoride is fed to an acetylation reactor 6 equipped with a stirrer 19 and is contacted there with stirring with the starting aromatic compound fed through a pipe 7 and with hydrogen fluoride fed through a pipe 8. The reaction begins in two liquid phases of the hydrogen fluoride phase and the starting material oil phase, which then change into a homogeneous liquid phase as the reaction proceeds. The reaction liquid is drawn out through a pipe 9, led to a hydrogen fluoride recovery column 10, and contacted there with a diluent such as benzene which is being refluxed and recycled. Hydrogen fluoride is separated by vaporization and drawn out through a pipe 11. The column top vapor is condensed by cooling and separated into layers. The benzene phase is refluxed from a pipe 12 to the recovery column 10; hydrogen fluoride is recycled to the acetyl fluoride generating apparatus and the acetylation reactor (not shown in the Figure). From the bottom of the hydrogen fluoride recovery column is withdrawn through a pipe 13 the acetylation product, a crude product, whicn is then freed from trace amount of residual acid in a neutralization and washing equipment 14 and distilled in a distillation apparatus 15, whereby the byproduct is removed through a pipe 16, the unreacted raw material is removed through a pipe 17 to be recycled to the reaction step, and the final product is obtained through a pipe 18.

A suitable solvent is used to make the reaction proceed smoothly as required. This is added to the starting material in a pipe 7. Fig. 1 shows a case where the solvent and the diluent for decomposition are the same. But, in the case where both are different, further a process for recovering the solvent is added to it.

This invention will be further explained in detail below with reference to Examples, but it is not limited thereto.

Example 1

Synthesis of acetyl fluoride

A stainless steel packed column having a diameter of 50 mm and a height of 1000 mm provided with a top reflux apparatus and a bottom reboiler was used as the acetyl fluoride synthesizer. Acetic anhydride (22.0 moles per hour) and hydrogen fluoride (21.0 moles per hour) were mixed and fed continuously to the middle plate of the packed column, and heat was supplied to the reboiler with an electric heater at a rate of 260 Kcal per hour. The synthesizer was operated under a pressure of 1.0 kg/cm$^2$G. While reflux was applied so as to keep the temperature of the column top at about 35$^0$C, acetyl fluoride was distilled out of the column top at a rate of 21 moles per hour, and a liquid mixture comprising 21 moles of acetic acid and 1 mole of acetic anhydride was withdrawn every hour from the bottom. The yield of acetyl fluoride relative to supplied hydrogen fluoride was quantitative.

Acetylation of 2-methylnaphthalene

Two stainless steel vessels each equipped with a stirrer, the first reactor having an inner liquid volume of 6ℓ and the second reactor having an inner liquid volume of 4ℓ , were connected in series to be used as the acetylation reactor. A solution comprising 1.5kg of 2-methylnaphthalene and 0.3kg of benzene was fed every hour to the first reactor. Simultaneously, 0.5kg per hour of acetyl fluoride synthesized above and 2.5kg per hour of hydrogen fluoride were also fed to the first reactor.

The reaction temperature was adjusted to 25$^0$C by passing cooling water through the jacket of the reactor. The pressure in the reaction was 1 kg/cm$^2$G. The reaction mixture was continuously withdrawn from the second reactor to be fed to the subsequent hydrogen fluoride recovery column.

Recovery of hydrogen fluoride

The packed column used in acetyl fluoride synthesis was employed as the hydrogen fluoride recovery column. The hydrogen fluoride recovery column was charged with benzene, and heat was supplied at a rate of 300 Kcal per hour to the reboiler under a pressure of 1 kg/cm²G to keep the benzene refluxing.

Then the above-mentioned reaction mixture was continuously fed to the upper part of the column at a rate of 1kg per hour, and the hydrogen fluoride recovery column was continuously operated while being replenished with benzene.

From the column top were distilled out hydrogen fluoride and unreacted acetyl fluoride, while from the column bottom were recovered every hour 243g of acetylated methylnaphthalene, 109g of unreacted methylnaphthalene, and 20g of a high boiling point product as the crude acetylation product. The acetylation product contained 75% of 2-acetyl-6-methylnaphthalene.

Example 2

Acetylation of toluene

Toluene was acetylated by using the same apparatus and the same operation as in Example 1.

To the first reactor were fed every hour 0.9kg of toluene, 0.4kg of acetyl fluoride, and 2.0kg of hydrogen fluoride, and the reaction was conducted at a reaction temperature of 40°C and under a reaction pressure of 1.5 kg/cm²G. The reaction mixture was continuously withdrawn from the second reactor and hydrogen fluoride was recovered in the same manner as in Example 1. The crude product obtained from the bottom of the hydrogen fluoride recovery column had the following composition: unreacted toluene 55%, methylacetophenone 41%, high boiling point product 4%. The methylacetophenone contained 97.5% of 4-methylacetophenone.

Example 3

Acetylation of m-xylene

A stainless steel autoclave of 500ml volume equipped with a jacket and a stirrer was used as the acetylation reactor.

A solution of 56g (0.9 mole) of acetyl fluoride in 103.1g (1 mole) of m-xylene was placed in the autoclave, and then, with cooling, 300g (15 moles) of hydrogen fluoride was introduced thereinto. The mixture was allowed to react at a reaction temperature of 40°C under a reaction pressure of 1.5 kg/cm²G for 1.5 hours. Thereafter, the reaction mixture was withdrawn into ice water. The resulting oil layer was washed with alkaline water and distilled to determine the amount of high boiling point byproducts and analyzed by gas chromatograph to determine the yield of the acetylated product.

Examples of acetylations conducted in the same manner as mentioned above using various aromatic compounds as the starting material are summarized in Table 1.

EP 0 215 351 B1

Table 1

| Starting aromatic compound (A) | Acetylation conditions | | | | Acetylation yield relative to (A) (mol %) |
|---|---|---|---|---|---|
| | Molar ratio | | Temperature (°C) | Time (hour) | |
| | ACF/(A) | HF/ACF | | | |
| 2-Methylnaphthalene | 0.75 | 16 | 25 | 2 | 60 |
| Toluene | 0.65 | 15 | 40 | 3 | 33 |
| m-Xylene | 0.9 | 15 | 40 | 1.5 | 75 |
| Mesitylene | 0.9 | 10 | 40 | 1.5 | 82 |
| Isobutylbenzene | 0.7 | 15 | 40 | 4 | 40 |
| Phenol | 0.9 | 20 | 25 | 3 | 82 |
| Anisole | 0.95 | 20 | 25 | 2 | 89 |
| Phenyl ether | 0.8 | 20 | 25 | 2 | 71 |

to be continued

Table 1 (cont'd)

| | Composition of acetylation product (Gas chromatographic analysis) | High-boiling byproduct relative to acetylation product (wt.%) |
|---|---|---|
| 2-Acetyl-6-methylnaphthalene | 75% | 8 |
| 4-Methylacetophenone | 97.5% | 10 |
| 2,4-Dimethylacetophenone | 100% | 7 |
| 2,4,6-Trimethylacetophenone | 100% | 7 |
| p-Isobutylacetophenone | 98% | 4 |
| p-Hydroxyacetophenone | 96% | 7 |
| p-Methoxyacetophenone | 97% | 6 |
| p-Phenoxyacetophenone | 98% | 9 |

Claims

1. A continuous process for producing an acetyl-substituted aromatic compound which comprise:

obtaining an acetyl fluoride by the reaction of acetic anhydride with a substantially anhydrous hydrogen fluoride and separating formed acetyl fluoride by distillation,

making an aromatic compound selected from alkylbenzenes, alkyl-naphtalenes, phenols, naphtols or aromatic ethers react with the separated acetyl fluoride in the presence of substantially anhydrous hydrogen fluoride as a catalyst at a temperature of from 0°C - 70°C,

thermally decomposing the resulting complex compound between the acetyl-substituted aromatic

7

compound and hydrogen fluoride at a temperature of 40°C or higher
**characterized in that** acetyl fluoride is obtained by making the molar ratio of acetyl fluoride to starting aromatic compound 1 or below, and making an excess of acetic anhydride of 5 mol % or below react with hydrogen fluoride.

2. A process for according to claim 1, **characterized in that** the reaction pressure in the acetylation is from atmospheric pressure to 2 kg/cm$^2$ (20265 Pa)G.

3. A process according to claim 1, **characterized in that** the thermal decomposition of the complex compound between the acetyl-substituted aromatic compound and hydrogen fluoride is performed in the presence of an inert solvent.

### Revendications

1. Un procédé continu pour la préparation d'un composé aromatique acétyl-substitué, qui comprend:
   l'obtention d'un fluorure d'acétyle par la réaction de l'anhydride acétique avec un fluorure d'hydrogène sensiblement anhydre, et la séparation par distillation du fluorure d'acétyle formé,
   la mise en réaction d'un composé aromatique choisi parmi les alcoylbenzènes, les alcoylnaphtalènes, les phénols, les naphtols ou les éthers aromatiques avec le fluorure d'acétyle séparé en présence de fluorure d'hydrogène sensiblement anhydre comme catalyseur à une température de 0°C à 70°C,
   la décomposition thermique du composé complexe formé entre le composé aromatique acétyl-substitué et le fluorure d'hydrogène à une température de 40°C ou plus,
   caractérisé en ce que le fluorure d'acétyle est obtenu par fixation du rapport molaire du fluorure d'acétyle au composé aromatique de départ à 1 ou moins et mise en réaction d'un excès d'anhydride acétique de 5% molaires ou moins avec du fluorure d'hydrogène.

2. Un procédé selon la revendication 1, caractérisé en ce que la pression de réaction dans l'acétylation est comprise entre la pression atmosphérique et 2 kg/cm$^2$ (20 265 Pa) manométriques.

3. Un procédé selon la revendication 1, caractérisé en ce que la décomposition thermique du composé complexe formé entre le composé aromatique acétyl-substitué et le fluorure d'hydrogène est effectuée en présence d'un solvant inerte.

### Ansprüche

1. Kontinuierliches Verfahren zur Herstellung einer acetylsubstituierten aromatischen Verbindung, umfassend:
   Erzielung eines Acetylfluorids durch die Umsetzung von Essigsäureanhydrid mit einem im wesentlichen wasserfreien Wasserstofffluorid und Abtrennen des gebildeten Acetylfluorids durch Destillation,
   Umsetzen einer aromatischen Verbindung, gewählt aus Alkylbenzolen, Alkylnaphthalinen, Phenolen, Naphtholen oder aromatischen Ethern mit dem abgetrennten Acetylfluorid in Gegenwart von im wesentlichen wasserfreiem Wasserstofffluorid als Katalysator bei einer Temperatur von 0°C - 70°C,
   thermisches Zersetzen der resultierenden Komplex-Verbindung zwischen der acetylsubstituierten aromatischen Verbindung und Wasserstofffluorid bei einer Temperatur von 40°C oder darüber,
   dadurch gekennzeichnet, daß Acetylfluorid dadurch erzielt wird, daß das molare Verhältnis von Acetylfluorid zu der aromatischen Ausgangsverbindung auf 1 oder darunter eingestellt wird, und daß ein Überschuß an Essigsäureanhydrid von 5 Mol % oder darunter mit dem Wasserstofffluorid umgesetzt wird.

2. Verfahren nach spruch 1, dadurch gekennzeichnet, daß der Reaktionsdruck bei der Acetylierung im Bereich von atmosphärischem Druck bis 2 kg/cm$^2$ (20265 Pa)G liegt.

3. Verfahren nach Anspruch 1, dadurch gekennzeichnet, daß die thermische Zersetzung der Komplex-Verbindung zwischen der acetylsubstituierten aromatischen Verbindung und Wasserstofffluorid in Gegenwart eines inerten Lösungsmittels durchgeführt wird.

# F I G . I